# EUROPÄISCHE PATENTSCHRIFT

(11) **EP 2 516 342 B1**
(45) Veröffentlichungstag und Bekanntmachung des Hinweises auf die Patenterteilung: **18.05.2016**
(21) Anmeldenummer: 10796296.1
(22) Anmeldetag: 23.12.2010
(51) Int. Cl.: C03C 10/00, A61C 13/00, C03B 32/02, C04B 41/00, A61K 6/02, A61K 6/027

(54) **LITHIUMDISILIKAT-GLASKERAMIK, VERFAHREN ZU DEREN HERSTELLUNG UND DEREN VERWENDUNG**
LITHIUM DISILICATE GLASS CERAMICS, METHOD FOR THE PRODUCTION THEREOF AND USE THEREOF
VITROCÉRAMIQUE RENFERMANT DU DISILICATE DE LITHIUM, SON PROCÉDÉ DE PRODUCTION ET SON UTILISATION

(30) Priorität: 23.12.2009 DE 102009060274
(43) Veröffentlichungstag der Anmeldung: 31.10.2012
(73) Patentinhaber: Fraunhofer-Gesellschaft zur Förderung der angewandten Forschung e.V., 80686 München (DE); Vita Zahnfabrik H. Rauter GmbH & Co. KG, 79713 Bad Säckingen (DE); DeguDent GmbH, 63457 Hanau (DE)
(72) Erfinder: DURSCHANG, Bernhard, 97228 Rottendorf (DE); PROBST, Jörn, 92273 Kürnach (DE); THIEL, Norbert, 79713 Bad Säckingen (DE); BIBUS, Joachim, 79713 Bad Säckingen (DE); VOLLMANN, Markus, 63571 Gelnhausen (DE); SCHUSSER, Udo, 63755 Alzenau (DE)
(74) Vertreter: Pfenning, Meinig & Partner mbB
(86) Internationale Anmeldenummer: PCT/EP2010/007918
(87) Internationale Veröffentlichungsnummer: WO 2011/076422

(56) Entgegenhaltungen:
- EP-A1- 1 505 041
- EP-A1- 2 377 831
- DE-A1-102010 050 275
- US-A1- 2007 042 889
- OLIVEIRA DE A P N ET AL: "SINTERING AND CRYSTALLIZATION OF A GLASS POWDER IN THE LI2O-ZRO2- SIO2 SYSTEM", JOURNAL OF THE AMERICAN CERAMIC SOCIETY, BLACKWELL PUBLISHING, MALDEN, MA, US, Bd. 81, Nr. 3, 1. März 1998 (1998-03-01), Seiten 777-780, XP000738310, ISSN: 0002-7820
- MONTEDO ET AL: "Low thermal expansion sintered LZSA glass-ceramics", AMERICAN CERAMIC SOCIETY BULLETIN, AMERICAN CERAMIC SOCIETY. COLUMBUS, US, Bd. 87, Nr. 7, 1. Januar 2008 (2008-01-01) , Seiten 34-40, XP001537398, ISSN: 0002-7812

## Beschreibung

Die Erfindung betrifft Glaskeramiken auf Basis des Lithiumdisilikat-Systems, die in einer Zwischenstufe der Kristallisation einfach mechanisch bearbeitbar sind und nach der vollständigen Kristallisation eine hochfeste, hoch transluzente und chemisch stabile Glaskeramik darstellen. Ebenso betrifft die Erfindung ein Verfahren zur Herstellung dieser Glaskeramiken. Verwendung finden die erfindungsgemäßen Glaskeramiken als Dentalmaterial.

Lithiumdisilikat-Glaskeramiken sind aus der Literatur gut bekannt und mehrere Patente basieren auf diesem Glaskeramiksystem. So werden z.B. in der EP-B-536 479 selbstglasierte Lithiumdisilikat-Glaskeramik-Gegenstände für die Herstellung von Tafelgeschirr beschrieben, in der EP-B-536 572 Lithiumdisilikat-Glaskeramiken, die durch das Aufstreuen eines feinteiligen gefärbten Glases auf ihre Oberfläche als Auskleidungselemente für Bauzwecke eingesetzt werden können.

Ein Schwerpunkt der patentierten Lithiumdisilikatglaskeramiken liegt-in dentalen Anwendungen. Dies liegt daran, dass die Kristallisation der Lithiumdisilikatkristalle über eine Phase geringerer Festigkeit (Lithiummetasilicat) erfolgt und das Materialsystem dadurch u.a. Chairside-Verfahren zugänglich ist (siehe S.D. Stookey: "Chemical Machining of Photosensitive Glass", Ind. Eng. Chem., 45, S. 115-118 (1993) and S.D. Stookey: "Photosensitively Opacifiable Glass" US-A-2,684,911 (1954)). Untersuchung von Borom, z.B. M.-P. Borom, A.M. Turkalo, R.H. Doremus: "Strength and Microstructure in Lithium Disilicate Glass-Ceramics", J. Am. Ceream. Soc., 58, Nr. 9-10, S. 385-391 (1975) und M.-P. Borom, A.M. Turkalo, R.H. Doremus: "Verfahren zum Herstellen von Glaskeramiken" DE-A-24 51 121 (1974) zeigen, dass Glaskeramiken, die Lithiummetasilikat als Hauptphase enthalten, eine verringerte Festigkeit im Vergleich zu Glaskeramiken haben, die Lithiumdisilikat als einzige kristalline Phase enthalten.

Dieses Prinzip wurde ausgenutzt, um in einem zweistufigen Kristallisations-prozess zuerst eine Glaskeramik herzustellen, die mechanisch gut bearbeitbar ist, z.B. mittels CAD/CAM-Verfahren, und diese anschließend in einer zweiten Kristallisationsstufe zur Dentalglaskeramik zu prozessieren. Dieses Verfahren ist geeignet, um dentale Restaurationen nach dem sog. chair-side-Verfahren verwenden zu können. Bei diesem Verfahren wird in der Zahnarztpraxis aus einem Glaskeramikblock nach der ersten Kristallisationsstufe mittels CAD/CAM eine individuell angepasste Krone/Onlay/Inlay herausgefräst, diese in einem Spezialofen der zweiten Kristallisationsstufe unterzogen und direkt in der ersten und einzigen Zahnarztsitzung dem Patienten eingesetzt (DE 10 2005 028 637).

Außerdem wurden in WO-A-95/32678 und US-A-5,507,981 Lithiumdisilikat-Glaskeramiken beschrieben, die durch den Einsatz eines speziellen verpressbaren Tiegels durch Heißpressen zu geformten Dentalprodukten verarbeitet werden können. Weiter sind aus der DE-C-14 21 886 Glaskeramiken auf Basis von SiO₂ und Li₂O bekannt, welche große Mengen an physiologisch sehr bedenklichem Arsenoxid enthalten. Auch in US-A-4,515,634 und in FR-A-2 655 264 werden zur Herstellung von Dentalkronen und -brücken geeignete Lithiumdisilikat-Glaskeramiken offenbart.

Aus OLIVERA DE A P N ET AL: "SINTERING AND CRYSTALLIZATION OF A GLASS POWER IN THE LI2O-ZRO2-SIO2 SYSTEM", JOURNAL OF THE AMERICAN CERAMIC SOCIETY; BLACKWELL PUBLISHING, MALDEN, MA, US, Bd. 81, Nr. 3, 1. März 1998 (1998-03-01), Seiten 777-780, XP000738310, ISSN: 0002-7820 und MONTEDO ET AL: "Low thermal expansion sintered LZSA glass ceramics", AMERICAN CERAMIC SOCIETY BULLETIN, AMERICAN CERAMIC SOCIETY BULLETIN, AMERICAN CERAMIC SOCIETY, COLUMBUS, US, Bd. 87, Nr. 7, 1. Januar 2008 (2008-01-01), Seiten 34-40, XP001537398, ISSN: 0002-7812 sind Lithiumdisilicat-Glaskeramiken auf Zirkon-Basis bekannt.

Aus US 2007/0042889 ist eine Lithiumsilikat-Glaskeramik bekannt, die einfach maschinell bearbeitet werden kann und dann in dentale Produkte mit hoher Festigkeit geformt werden kann.

Alle bekannten Lithiumdisilikat-Glaskeramiken zeigen Unzulänglichkeiten bei ihrer Verarbeitung zu geformten Produkten und/oder mechanischen bzw. optischen Eigenschaften und/oder chemischen Stabilität. Insbesondere sind beim Einsatz im Dentalbereich für alle genannten Eigenschaften gleichermaßen hohe Ansprüche zu erfüllen.

Ausgehend hiervon war es Aufgabe der vorliegenden Erfindung, eine Glaskeramik bereitzustellen, die verbesserte mechanische und optische Eigenschaften sowie eine verbesserte chemische Stabilität gegenüber den aus dem Stand der Technik bekannten (Glas-)Keramiken aufweist.

Diese Aufgabe wird durch die Lithiumdisilikat-Glaskeramik mit den Merkmalen des Anspruchs 1, sowie durch das Verfahren zur Herstellung dieser Glaskeramik mit den Merkmalen des Anspruchs 6 gelöst. In Anspruch 9 werden erfindungsgemäße Verwendungen angegeben. Ebenso wird ein geformtes Dentalprodukt mit den Merkmalen des Anspruchs 10 bereitgestellt. Die weiteren abhängigen Ansprüche zeigen vorteilhafte Weiterbildungen auf.

Im Rahmen der vorliegenden Erfindung wurden Glaszusammensetzungen entwickelt, die sich in einem zweistufigen Herstellungsprozess darstellen lassen, nach der ersten Kristallisationsstufe, insbesondere mittels CAD/CAM gut zu bearbeiten sind und nach einer sehr kurzen zweiten Kristallisationsstufe sowohl hochtransparent und hochfest sind als auch bessere chemische Beständigkeiten aufweisen, als die bekannten Lithiumdisilikat-Glaskeramiken.

Überraschenderweise hat sich gezeigt, dass die Zugabe von ZrO₂zu bestimmten Glaszusammensetzungen zu Glaskeramiken führt, die in einer Kristallisationszwischenstufe sehr gut bearbeitbar sind und im Endzustand hervorragende Festigkeitswerte, außerordentliche Transluzenz und deutlich erhöhte chemische Beständigkeiten aufweisen.

Es zeigte sich, dass sich bis zu 20 Gew.-% eines Stabilisators ausgewählt aus der Gruppe bestehend aus ZrO₂ oder Mischungen von ZrO₂ und HfO₂, in das Glas einbauen lässt, ohne die Struktur signifikant zu beeinflussen. Entgegen allen Erwartungen kristallisiert der Stabilisator hierbei nicht als eigene Kristallphase aus, sondern verbleibt in der Restglasphase. Durch den hohen Anteil in der amorphen Phase werden in dieser Phase die mechanischen und chemischen Beständigkeiten enorm verbessert, was auch zu verbesserten Eigenschaften im Endprodukt führt.

Insbesondere die chemische Beständigkeit lässt sich über die Zusammensetzung der Restglasphase verbessern, da die Glasphase eine deutliche höhere Löslichkeit aufweist als das Lithiumdisilikat und somit die Schwachstelle bezüglich chemischem Angriff darstellt. Die extrem hohe Löslichkeit des Stabilisators (ZrO₂) in der Glasphase ist insbesondere beachtenswert, da z.B. Zirkonoxid bei vielen silikatischen Glaskeramiken als Keimbildner fungiert, d.h. bei einer Temperaturbehandlung als erste Phase auskristallisiert und an diesen ZrO₂-Kristallen sich die eigentlich angestrebte Kristallphase erleichtert und feinkristallin ausscheidet.

Durch die hohen Anteile des Stabilisators, die im Wesentlichen in der amorphen Phase verbleiben, ist der kristalline Anteil entsprechend begrenzt. Hierdurch und durch die geringe Kristallitgröße der Lithiumdisilikatkristalle entsteht eine gute Transluzenz der Materialien nach der zweiten Kristallisation. Die Transluzenz wird jedoch auch noch dadurch verbessert, dass der Brechungsindex der Glasphase wiederum durch den Stabilisator erhöht wird und sich dadurch dem Brechungsindex des Lithiumdisilikats anpasst. Bei Glaskeramiken, bei denen der Brechungsindex der amorphen Matrixphase mit dem Brechungsindex der kristallinen Phase/Phasen übereinstimmt, findet man sehr gute Transluzenzeigenschaften, relativ unabhängig von der Kristallitgröße. In den erfindungsgemäßen Glaskeramiken sind also alle drei Punkte zur Erzeugung einer höchst transluzenten Glaskeramik erfüllt:
- limitierter Kristallphasenanteil,
- kleine Kristalle (< 500 nm),
- angepasster Brechungsindex von amorpher und kristalliner Phase.
Der hohe Anteil des Stabilisators wirkt sich in der Glaskeramik also in
- einer verbesserten chemischen Beständigkeit,
- höheren Festigkeitswerten und
- einer in mehrerer Hinsicht verbesserten Transluzenz
zu entsprechenden Glaskeramiken ohne bzw. mit geringem ZrO₂- oder HfO₂-Anteil aus.

Die erfindungsgemäßen Glaskeramiken können vorzugsweise mittels eines Verfahrens hergestellt werden, bei dem
(a) ein Ausgangsglas hergestellt wird, das die Komponenten der Glaskeramik enthält,
(b) das Ausgangsglas einer ersten Wärmebehandlung bei einer ersten Temperatur unterworfen wird, um eine Glaskeramik herzustellen, welche Lithiummetasilikat als einzige bzw. Hauptkristallphase aufweist und
(c) diese Glaskeramik einer zweiten Wärmebehandlung unterzogen wird, bei der das Lithiummetasilikat mit SiO₂ aus der Glasphase in Lithiumdisilikat umgewandelt wird und anschließend Lithiumdisilikat als einzige oder Hauptkristallphase vorliegt.

Die Kristallisation zum Lithiummetasilikat findet vorzugsweise bei Temperaturen zwischen 620 °C und 800 °C statt, mit Zeiten zwischen 1 und 200 Minuten, bevorzugt zwischen 650 °C und 750 °C für 10 bis 60 Minuten.

Die Kristallisation zum Lithiumdisilikat findet vorzugsweise bei Temperaturen zwischen 800 °C und 1040 °C statt, mit Zeiten von 5 bis 200 Minuten, bevorzugt zwischen 800 °C und 870 °C für 5 bis 30 Minuten.

Anhand der nachfolgenden Beispiele soll der erfindungsgemäße Gegenstand näher erläutert werden, ohne diesen auf die hier gezeigten speziellen Ausführungsformen einschränken zu wollen.

### Beispiele 1 bis 6

In den Beispielen 1 bis 6 sind Zusammensetzungen von hochzirkonoxidhaltigen Gläsern angegeben, die durch eine zweistufige Temperaturbehandlung erst zu gut mechanisch bearbeitbaren Lithiummetasilicat-Glaskeramiken und anschließend zu hoch-transluzenten, hoch-festen und chemisch stabilen Lithiumdisilicat-Glaskeramiken überführt werden.

Die Zusammensetzungen mit ihren Bestandteilen sind in Tabelle 1 dargestellt.

**Tabelle 1**

| | **B1** | **B2** | **B3** | **B4** | **B5** | **B6** |
|---|---|---|---|---|---|---|
| SiO₂ | 66,9 | 65,8 | 65,5 | 63,7 | 63,5 | 63,5 |
| Li₂O | 13,9 | 13,7 | 13,6 | 13,2 | 14,4 | 12,9 |
| ZrO₂ | 10,0 | 10,0 | 12,0 | 11,7 | 12,7 | 13,5 |
| Al₂O₃ | 3,2 | 3,1 | 3,1 | 3,0 | 3,3 | 3,5 |
| P₂O₅ | 3,0 | 3,0 | 3,0 | 2,9 | 3,1 | 3,4 |
| K₂O | 2,9 | 2,9 | 2,9 | 2,8 | 3,0 | 3,2 |
| CeO₂ | - | 1,0 | - | 2,0 | - | - |
| Er₂O₃ | - | 0,2 | - | 0,3 | - | - |
| Tb₂O₃ | - | 0,3 | - | 0,3 | - | - |

Die Gläser wurden bei 1500 °C erschmolzen und in Metallformen zu Blocks gegossen. Die Blocks wurden bei 560 °C im Ofen entspannt und langsam abgekühlt. Für die unterschiedlichen Charakterisierungsverfahren wurden die Glasblocks zerteilt und einer ersten Kristallisationsbehandlung unterzogen. Hierfür wurden die Gläser für 10 bis 120 Minuten bei 600 °C bis 750 °C ausgelagert. Hierdurch wurden Glaskeramiken mit Festigkeitswerten von 150 MPa bis 220 MPa hergestellt. Als Kristallphase wurde hierbei ausschließlich Lithiummetasilicat festgestellt. In diesem Zustand ist eine Bearbeitung mittels CAD/CAM-Methoden sehr gut möglich.

Mit einer zweiten kurzen Kristallisation bei 800 °C bis 950 °C für 3 bis 15 Minuten findet eine Umkristallisation des Lithiummetasilikates mit amorphem SiO₂ aus der Glasphase zum Lithiumdisilikat statt und es kommt zu einer Festigkeitserhöhung auf 300 MPa bis 450 MPa. Neben der Lithiumdisilikatphase kann hierbei eine zirkonoxidhaltige Nebenkristallphase entstehen. Außerdem können noch geringe Reste von Lithiummetasilikat vorliegen. Die eindeutige Hauptkristallphase ist das Lithiumdisilikat.

In Tabelle 2 sind die Kristallisationsbedingungen von einzelnen Gläsern sowie die entstehenden Kristallphasen und Festigkeitswerte dargestellt.

**Tabelle 2**

| **Glas** | **B1** | **B2** | **B3** | **B4** | **B5** | **B6** |
|---|---|---|---|---|---|---|
| 1. Kristallisation | 650 °C | 700 °C | 650 °C | 700 °C | 700 °C | 700 °C |
| | 20 min | 40 min | 30 min | 20 min | 40 min | 40 min |
| 2. Kristallisation | 850 °C | 830 °C | 870 °C , | 850 °C | 820 °C | 830 °C |
| | 10 min | 10 min | 20 min | 8 min | 10 min | 10 min |
| Kristallphasen | | | | | | |
| - Hauptphase (> 80 %) | Disilikat | Disilikat | Disilikat | Disilikat | Disilikat | Disilikat |
| - Nebenphase (< 20 %) | - | - | - | - | Metasilikat | Metasilikat |
| Transluzenz | hervorragend | sehr gut | hervorragend | sehr gut | hervorragend | hervorragend |
| 3-Punkt-Biegebr.-Festigkeit | 375 MPa | 413 MPa | 380 MPa | 418 MPa | 356 MPa | 385 MPa |

## Patentansprüche

1. Lithiumdisilikat-Glaskeramik mit folgender Zusammensetzung:
55 bis 70 Gew.-% SiO₂,
10 bis 15 Gew.-% Li₂O,
10 bis 20 Gew.-% des Stabilisators ausgewählt aus der Gruppe bestehend aus ZrO₂ oder Mischungen von ZrO₂ und HfO₂,
0,1 bis 5 Gew.-% K₂O,
0,1 bis 5 Gew.-% Al₂O₃,
0 bis 10 Gew.-% an Zusatzstoffen ausgewählt aus der Gruppe bestehend aus Boroxid, Phosphoroxid, Fluor, Natriumoxid, Bariumoxid, Strontiumoxid, Magnesiumoxid, Zinkoxid, Calciumoxid, Yttriumoxid, Titanoxid, Nioboxid, Tantaloxid, Lanthanoxid und Mischungen hiervon sowie
0 bis 10 Gew.-% an Farbstoffen,
wobei der Stabilisator im Wesentlichen in der amorphen Phase vorliegt.

2. Lithiumdisilikat-Glaskeramik nach Anspruch 1,
**dadurch gekennzeichnet, dass** die Farbstoffe glasfärbende Oxide und/oder Farbkörper sind.

3. Lithiumdisilikat-Glaskeramik nach einem der vorhergehenden Ansprüche,
**dadurch gekennzeichnet, dass** die glasfärbenden Oxide ausgewählt sind aus der Gruppe der Oxide von Eisen, Titan, Cer, Kupfer, Chrom, Kobalt, Nickel, Mangan, Selen, Silber, Indium, Gold, Seltenerdmetallen, insbesondere Neodym, Praseodym, Samarium und Europium.

4. Lithiumdisilikat-Glaskeramik nach einem der vorhergehenden Ansprüche,
**dadurch gekennzeichnet, dass** die Farbkörper dotierte Spinelle sind.

5. Lithiumdisilikat-Glaskeramik nach einem der vorhergehenden Ansprüche mit folgender Zusammensetzung:
58 bis 64 Gew.-% SiO₂,
11 bis 13 Gew.-% Li₂O,
10 bis 15 Gew.-% des Stabilisators ausgewählt aus der Gruppe bestehend aus ZrO₂, HfO₂ oder Mischungen hiervon,
2 bis 5 Gew.-% K₂O,
2 bis 5 Gew.-% Al₂O₃,
2 bis 5 Gew.-% P₂O₅ sowie
0 bis 5 Gew.-% an Zusatzstoffen ausgewählt aus der Gruppe bestehend aus Boroxid, Phosphoroxid, Fluor, Natriumoxid, Bariumoxid, Strontiumoxid, Magnesiumoxid, Zinkoxid, Calciumoxid, Yttriumoxid, Titanoxid, Nioboxid, Tantaloxid, Lanthanoxid und Mischungen hiervon sowie
0 bis 10 Gew.-% an Farbstoffen.

6. Verfahren zur Herstellung einer Lithiumdisilikat-Glaskeramik nach einem der vorhergehenden Ansprüche, bei dem
a) ein Ausgangsglas hergestellt wird, das die Komponenten der Glaskeramik enthält,
b) das Ausgangsglas einer ersten Wärmebehandlung zur Herstellung einer Glaskeramik, die Lithiummetasilicat als Hauptkristallphase aufweist, unterworfen wird,
c) die Glaskeramik aus b) einer zweiten Wärmebehandlung unterworfen wird, bei der das Lithiummetasilicat mit SiO₂ aus der Glasphase in Lithiumdisilicat umgewandelt wird und anschließend Lithiumdisilicat als Hauptkristallphase vorliegt.

7. Verfahren nach Anspruch 6,
**dadurch gekennzeichnet, dass** die erste Wärmebehandlung bei einer Temperatur von 620 °C bis 800 °C über einen Zeitraum von 1 bis 200 min, insbesondere von 650 °C bis 750 °C über einen Zeitraum von 10 bis 60 min, erfolgt.

8. Verfahren nach einem der Ansprüche 6 oder 7,
**dadurch gekennzeichnet, dass** die zweite Wärmebehandlung bei einer Temperatur von 800 °C bis 1040 °C über einen Zeitraum von 5 bis 200 min, insbesondere von 800 °C bis 870 °C über einen Zeitraum von 5 bis 30 min erfolgt.

9. Verwendung der Lithiumdisilikat-Glaskeramik nach einem der Ansprüche 1 bis 5 als Dentalmaterial oder als Komponente eines Dentalmaterials.

10. Geformtes Dentalprodukt enthaltend eine Lithium-disilikat-Glaskeramik nach einem der Ansprüche 1 bis 5, insbesondere in Form eines Inlays, eines Onlays, einer Brücke, eines Stiftaufbaus, einer Verblendung, einer (Teil)krone.

## Claims

1. Lithium disilicate glass ceramic having the following composition:
55 to 70% by weight SiO₂,
10 to 15% by weight Li₂O,
10 to 20% by weight of stabiliser selected from the group consisting of ZrO₂ or mixtures of ZrO₂ and HfO₂,
0.1 to 5% by weight K₂O,
0.1 to 5% by weight Al₂O₃,
0 to 10% by weight of additives selected from the group consisting of boron oxide, phosphorus oxide, fluorine, sodium oxide, barium oxide, strontium oxide, magnesium oxide, zinc oxide, calcium oxide, yttrium oxide, titanium oxide, niobium oxide, tantalum oxide, lanthanum oxide and mixtures thereof, as well as
0 to 10% by weight of dyes,
wherein the stabiliser is substantially present in the amorphous phase.

2. Lithium disilicate glass ceramic according to claim 1, **characterised in that** the dyes are glass-colouring oxides and/or pigments.

3. Lithium disilicate glass ceramic according to one of the preceding claims,
**characterised in that** the glass-colouring oxides are selected from the group of oxides of iron, titanium, cerium, copper, chromium, cobalt, nickel, manganese, selenium, silver, indium, gold, rare earth metals, in particular neodymium, praseodymium, samarium and europium.

4. Lithium disilicate glass ceramic according to one of the preceding claims,
**characterised in that** the pigments are doped spinels.

5. Lithium disilicate glass ceramic according to one of the preceding claims, having the following composition: 58 to 64% by weight SiO₂,
11 to 13% by weight Li₂O,
10 to 15% by weight of stabiliser selected from the group consisting of ZrO₂, HfO₂ or mixtures thereof,
2 to 5% by weight K₂O,
2 to 5% by weight Al₂O₃,
2 to 5% by weight P₂O₅ as well as
0 to 5% by weight of additives selected from the group consisting of boron oxide, phosphorus oxide, fluorine, sodium oxide, barium oxide, strontium oxide, magnesium oxide, zinc oxide, calcium oxide, yttrium oxide, titanium oxide, niobium oxide, tantalum oxide, lanthanum oxide and mixtures thereof, as well as
0 to 10% by weight of dyes.

6. Method for the production of a lithium disilicate glass ceramic according to one of the preceding claims, in which
a) an initial glass is produced which contains the components of the glass ceramic,
b) the initial glass undergoes a first heat treatment for the production of a glass ceramic which has lithium metasilicate as the main crystal phase,
c) the glass ceramic from b) undergoes a second heat treatment in which the lithium metasilicate is converted into lithium disilicate with SiO₂ from the glass phase and lithium disilicate is then present as the main crystal phase.

7. Method according to claim 6,
**characterised in that** the first heat treatment occurs at a temperature of 620°C to 800°C over a period of 1 to 200 minutes, in particular of 650°C to 750°C over a period of 10 to 60 minutes.

8. Method according to one of claims 6 or 7,
**characterised in that** the second heat treatment occurs at a temperature of 800°C to 1040°C over a period of 5 to 200 minutes, in particular of 800°C to 870°C over a period of 5 to 30 minutes.

9. Use of the lithium disilicate glass ceramic according to one of claims 1 to 5 as a dental material or as a component of a dental material.

10. Moulded dental product containing a lithium disilicate glass ceramic according to one of claims 1 to 5, in particular in the form of an inlay, an onlay, a bridge, a post and core, a veneer, a (partial) crown.

## Revendications

1. Vitrocéramique au disilicate de lithium possédant la composition suivante :
55 à 70 % en poids de SiO₂,
10 à 15 % en poids de Li₂O,
10 à 20 % en poids d'agent stabilisateur choisi dans le groupe constitué par le ZrO₂ ou des mélanges de ZrO₂ et de HfO₂,
0,1 à 5 % en poids de K₂O,
0,1 à 5 % en poids d'Al₂O3,
0 à 10 % en poids d'additifs choisis dans le groupe constitué par l'oxyde de bore, l'oxyde de phosphore, le fluor, l'oxyde de sodium, l'oxyde de baryum, l'oxyde de strontium, l'oxyde de magnésium, l'oxyde de zinc, l'oxyde de calcium, l'oxyde d'yttrium, l'oxyde de titane, l'oxyde de niobium, l'oxyde de tantale, l'oxyde de lanthane et des mélanges de ceux-ci, ainsi que
0 à 10 % en poids de colorants,
l'agent stabilisateur étant essentiellement présent en phase amorphe.

2. Vitrocéramique au disilicate de lithium selon la revendication 1, **caractérisée en ce que** les colorants sont des oxydes et/ou des corps colorants qui teintent le verre.

3. Vitrocéramique au disilicate de lithium selon l'une des revendications précédentes, **caractérisée en ce que** les oxydes qui teintent le verre sont choisis dans le groupe des oxydes de fer, titane, cérium, cuivre, chrome, cobalt, nickel, manganèse, sélénium, argent, indium, or, terres rares, en particulier néodyme, praséodyme, samarium et europium.

4. Vitrocéramique au disilicate de lithium selon l'une des revendications précédentes, **caractérisée en ce que** les corps colorants sont des aiguilles dopées.

5. Vitrocéramique au disilicate de lithium selon l'une des revendications précédentes, possédant la composition suivante :
58 à 64 % en poids de SiO₂,
11 à 13 % en poids de Li₂O,
10 à 15 % en poids d'agent stabilisateur choisi dans le groupe constitué par le ZrO₂, le HfO₂ou des mélanges de ceux-ci,
2 à 5 % en poids de K₂O,
2 à 5 % en poids d'Al₂O3,
2 à 5 % en poids de P₂O₅, ainsi que
0 à 5 % en poids d'additifs choisis dans le groupe constitué par l'oxyde de bore, l'oxyde de phosphore, le fluor, l'oxyde de sodium, l'oxyde de baryum, l'oxyde de strontium, l'oxyde de magnésium, l'oxyde de zinc, l'oxyde de calcium, l'oxyde d'yttrium, l'oxyde de titane, l'oxyde de niobium, l'oxyde de tantale, l'oxyde de lanthane et des mélanges de ceux-ci, ainsi que
0 à 10 % en poids de colorants.

6. Procédé de fabrication d'une vitrocéramique au disilicate de lithium selon l'une des revendications précédentes, dans lequel
a) on prépare un verre de départ qui contient les composants de la vitrocéramique,
b) on soumet le verre de départ à un premier traitement thermique afin d'obtenir une vitrocéramique qui contient du métasilicate de lithium en tant que phase cristalline principale,
c) on soumet la vitrocéramique obtenue à l'étape b) à un deuxième traitement thermique dans lequel le métasilicate de lithium est converti avec du SiO₂ à partir de la phase vitreuse en disilicate de lithium, suite à quoi le disilicate de lithium est présent en tant que phase cristalline principale.

7. Procédé selon la revendication 6, **caractérisé en ce que** le premier traitement thermique se fait à une température de 620 à 800 °C sur une durée de 1 à 200 minutes, en particulier à une température de 650 à 750 °C sur une durée de 10 à 60 minutes.

8. Procédé selon la revendication 6 ou la revendication 7, **caractérisé en ce que** le deuxième traitement thermique se fait à une température de 800 à 1040 °C sur une durée de 5 à 200 minutes, en particulier à une température de 800 °C à 870 °C sur une durée de 5 à 30 minutes.

9. Utilisation de la vitrocéramique au disilicate de lithium selon l'une des revendications 1 à 5 comme matériau dentaire ou comme composant d'un matériau dentaire.

10. Produit dentaire façonné contenant une vitrocéramique au disilicate de lithium selon l'une des revendications 1 à 5, en particulier sous la forme d'un inlay, d'un onlay, d'un bridge, d'un tenon radiculaire, d'une facette, d'une couronne complète ou partielle.
